(19) Europäisches Patentamt
European Patent Office
Office européen des brevets

(11) **EP 2 163 894 A1**

(12) **EUROPEAN PATENT APPLICATION**

(43) Date of publication:
**17.03.2010 Bulletin 2010/11**

(51) Int Cl.:
*G01N 33/50* (2006.01)    *G01N 33/574* (2006.01)

(21) Application number: **09168995.0**

(22) Date of filing: **31.08.2009**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(30) Priority: **02.09.2008   US 93594 P**

(72) Inventors:
• **Giuliano, Armando E.**
 **Los Angeles, CA 90049 (US)**
• **Hoon, Dave S.**
 **Los Angeles, CA 90025 (US)**
• **Hideki, Ishihara**
 **Kobe-shi Hyogo 651-0073 (JP)**

(74) Representative: **Paemen, Liesbet R.J. et al**
 **De Clercq, Brants & Partners**
 **E. Gevaertdreef 10 A**
 **9830 Sint-Martens-Latem (BE)**

(71) Applicants:
• **John Wayne Cancer Institute**
 **Santa Monica, CA 90404 (US)**
• **Sysmex Corporation**
 **Kobe-shi, Hyogo 651-0073 (JP)**

(54) **Method for predicting response of cancer patient to anticancer drug treatment**

(57)    A method is described for predicting the response of a cancer patient to an anticancer drug based on a cell cycle profile score obtained by analyzing a malignant tumor of the cancer patient, wherein the method can predict a disease outcome including a complete response, a partial response, stable disease, no response, and time to progression of disease as the patient's response to anticancer drug therapy.

FIG. 1

**Description**

FIELD OF THE INVENTION

The present invention relates to a method for predicting response of a cancer patient to anticancer drug as treatment.

BACKGROUND

As one of methods of cancer treatment, anticancer drug treatment is mentioned. However, pathological conditions of cancers are diversified depending on types of cancers, area of onset, or stage of cancer progression, and these diversifications and differences of responses of individual patients are responsible for the fact that there are many patients to whom anticancer drug treatment is not useful as well as patients to whom anticancer drug treatment is useful. Continued administrations of anticancer drugs to patients to whom anticancer drug treatment is not useful since this increases the side effects of the anticancer drugs thereby causing sufferings to patients.

For this reason, various methods for predicting sensitivities of anticancer drugs based on in vivo gene or protein expression have been investigated.

SUMMARY

The present invention is a method for predicting responses of patients to a chemotherapy or anti-cancer therapy which is useful as an index for judgment by physicians whether or not the specific treatment agent should be given to the patient. The major focus of the patent is to assess primary or metastatic tumors of patients prior to neoadjuvant therapy. The invention will allow determination whether a patient should receive treatment or not. This will avoid unnecessary treatment of patients in which the anticancer therapy is unlikely to be significantly effective. It will also allow screening of treatments of multiple drugs prior to use in patients using the assay. The uniqueness of the invention is its utility in predicting treatment outcome.

A first aspect of the present invention relates to a method for predicting a response of a cancer patient to an anticancer drug, including the steps of: acquiring or determining specific activities of cyclin-dependant kinase 1 (CDK1) and CDK2 contained in a malignant tumor obtained from a cancer patient; obtaining a cell cycle profile score based on the specific activity of CDK1 (CDK1 specific activity) and the specific activity of CDK2 (CDK2 specific activity); and predicting a degree of the response to the anticancer drug based on the cell cycle profile score, wherein the CDK1 specific activity is represented by a ratio of an activity value of CDK1 to an expression level of CDK1 and the CDK2 specific activity is represented by a ratio of an activity value of CDK2 to an expression level of CDK2. More particularly the degree of response to the anticancer drug treatment is predicted based on the cell cycle profile score prior to treatment.

The cell cycle profile score will be used to predict the patient's tumor (in vivo) response to the treatment. Specific embodiments of the methods according to all aspects of the invention will be aimed at neoadjuvant treatment of individuals with a known cancer comprising giving single or multiple drug combinations to a patient. Individual drug responses to the patients' tumor response will be assessed prior to and post neoadjuvant treatment. If the cell cycle profile score is higher (responsive rate is high; high being very responsive) then the patient is likely to benefit from treatment. If the patient score is low then the patient should not be given treatment.

A second aspect of the present invention relates to a method for predicting response of cancer patient to anticancer drug, including steps of: acquiring specific activities of cyclin-dependant kinase 1 (CDK1) and CDK2 contained in a malignant tumor obtained from a cancer patient; obtaining a cell cycle profile score (as described above) based on the specific activity of CDK1 (CDK1 specific activity) and the specific activity of CDK2 (CDK2 specific activity); and predicting a degree of tumor reduction based on the cell cycle profile score , wherein the CDK1 specific activity is represented by a ratio of an activity value of CDK1 to an expression level of CDK1 and the CDK2 specific activity is represented by a ratio of an activity value of CDK2 to an expression level of CDK2. More particularly the degree of tumor reduction is predicted based on the cell cycle profile score prior to treatment.

A third aspect of the present invention relates to a method for predicting a response of a cancer patient to anticancer drug, including steps of: acquiring specific activities of cyclin-dependant kinase 1 (CDK1) and CDK2 contained in a malignant tumor obtained from a cancer patient; obtaining a cell cycle profile score based on the specific activity of CDK1 (CDK1 specific activity) and the specific activity of CDK2 (CDK2 specific activity); and predicting a pathological complete response rate based on the cell cycle profile score, wherein the CDK1 specific activity is represented by a ratio of an activity value of CDK1 to an expression level of CDK1 and the CDK2 specific activity is represented by a ratio of an activity value of CDK2 to an expression level of CDK2. More particularly the pathological complete response is predicted based on the cell cycle profile score prior to treatment.

In specific embodiments, the methods according to the invention may be applied to multiple cancers such as melanoma, sarcoma, and breast, colorectal, gastric, lung, esophageal, pancreas, prostate, and other types carcinomas.

In particular embodiments, the methods according to the invention may be applied to any chemotherapy or anti-cancer treatment currently available or developed in the future. The invention also covers combination of anti-cancer drugs. Accordingly, in these particular embodiments, the predicting step in the methods according to the invention may be performed by predicting the degree of response to a combination of anticancer drugs.

A further aspect of the invention is to screen potential neoadjuvant treatment effectiveness as single and combination agents prior to neoadjuvant therapy.

According to particular embodiments, the methods according to the invention may be used for predicting a response to an anticancer drug treatment for neoadjuvant therapy.

According to particular embodiments, the methods according to the invention may further comprise the steps of performing a second acquiring step, a second obtaining step and a second predicting step post to the treatment.

According to particular embodiments, the methods according to the invention may be used to for predicting a response to an anticancer drug treatment for adjuvant therapy.

In certain embodiments of the methods of the invention, the cell cycle profile score is obtained based on the CDK1 specific activity and a ratio of the CDK2 specific activity to the CDK1 specific activity.

In specific embodiments of the methods of the invention, the cell cycle profile score is obtained based on the following expression:

$$\text{Cell cycle profile score} = F(x) \times G(y) \qquad (1)$$

wherein x represents the CDK1 specific activity, y represents the ratio of the CDK2 specific activity to the CDK1 specific activity.

In further particular embodiments of these methods F (x) and G (y) may be represented by the following expressions:

$$F(x)=a/(1+Exp(-(x-b)\times c))$$

$$G(y)=d/(1+Exp(-(y-e)\times f))$$

wherein a through f are constants.

BRIEF DESCRIPTION OF THE DRAWINGS

FIG. 1 is a histogram showing the relationship between specific activity of CDK1 obtained from a plurality of cancer patients and recurrence rate of the cancer.

FIG. 2 is a histogram showing the relationship between ratio of a specific activity of CDK1 and CDK2 obtained from a plurality of cancer patients and recurrence rate of the cancer.

FIG. 3 is a 3-dimensional graph using parametersof logarithm of specific activity of CDK1 (x-axis), logarithm of specific activity of CDK2 (y-axis), and cell cycle profile score C2PS (z-axis).

FIG. 4 is a graph in which cell cycle profile score C2PS (z-axis) shown in FIG. 3 is projected on 2-dimensional surface.

FIG. 5 is a graph showing relationship between cell cycle profile score C2PS and reduction rate of tumor.

FIG. 6 is a graph showing relationship between cell cycle profile score C2PS and frequency of patients with 50% tumor reduction rate.

FIG. 7 is a graph showing relationship between cell cycle profile score C2PS and frequency of patients with 80% tumor reduction rate.

FIG. 8 is a graph showing relationship between cell cycle profile score C2PS and pathological complete response (pCR) rate.

FIG. 9 is a block diagram showing the structure of the computer system 100.

FIG. 10 is a flow chart for performing an embodiment of the method executed by the computer system.

DETAILED DESCRIPTION OF THE EMBODIMENTS

The inventors have found that a disease outcome can be predicted on the basis of a cell cycle profile score obtained by analyzing a tumor of patient. The disease outcome includes a complete response, a partial response, stable disease, no response, and time to progression of disease as patient's response to anticancer drug therapy. The cell cycle profile score is obtained based on the relationship between CDK1 specific activity and CDK2 specific activity in the tumor sampled from the cancer patient. Here, CDK1 specific activity represents CDK1 activity value/CDK1 expression level, and CDK2 specific activity represents CDK2 activity value/CDK2 expression level. CDK specific activity is a parameter reflecting CDK enzyme activity per unit CDK protein amount contained in a sample. CDK specific activity is equivalent to the ratio of CDKs present in the cell, ratio of CDK showing activity and represents CDK activity level based on proliferative state of malignant tumor cells which are the judgment object.

Above-mentioned cancers include epithelial derived tumors, hematopoietic organ derived cancers, sarcomas, or the like. As for types of cancers, for example, breast cancer, gastric cancer, colorectal cancer, esophageal cancer, lung cancer, prostate cancer, leukemia, sarcomas, or the like are mentioned. By anticancer drug is meant chemical substances having antitumor efficacy for above-mentioned cancers. A malignant tumor sampled from a cancer patient means a tumor of a tissue where the control mechanism of proliferation is abnormal due to that, for example, harmony as an individual is spoiled in body tissue of the cancer patient.

In general, it may be considered that, since cancer cells proliferate actively exceeding normal proliferation control, when the ratio of cells in S-phase that is DNA replication phase and in G2-phase from completion of DNA synthesis to commencement of mitosis is high, the cells already became cancerous. Further, since it is considered that aneuploidy observed with cancer cells is caused when cells passed M-phase that is a cell division phase in abnormal state, or proceeded to G1-phase without passing M-phase, and then proceeded to S-phase without change. It may be considered that cells concerned are already cancerous also when ratio of cells existing in M-phase is low.

CDK1 is a protein exhibiting activity when proceeding from G2-phase to M-phase of the cell cycle, and CDK2 is a protein exhibiting activity when proceeding from G1-phase to S-phase of the cell cycle. Therefore, CDK specific activity ratio (CDK2 specific activity/CDK1 specific activity) is a numeric figure which reflects how much cells in S-phase or G2-phase are greater than cells in M-phase and can be used as a parameter accurately reflecting proliferation potency of cells.

CDK activity value is, for example, kinase activity level (unit is expressed by U) calculated from the amount of substrate being phosphorylated by CDK. As for the substrate which is phosphorylated by CDK, for example, histone H1 is cited for active type CDK1 and active type CDK2. CDK activity value can be measured by known CDK activity measuring method. Specifically, such a method is used that a sample containing active type CDK is prepared from cell culture of a measurement sample, substrate protein is caused to incorporate $^{32}P$ using the sample and $^{32}P$ labeled ATP ($\gamma$-[$^{32}P$]-ATP), amount of labeling of $^{32}P$ labeled phosphorylated substrate is measured, and is quantified based on a calibration curve prepared by a standard preparation. As for the method which does not use labeling of radioactive material, one described in US2002-0164673 is mentioned. According to this method, a sample containing target active type CDK is prepared from cell lysate of a specimen, adenosine 5'-O-(3-thiotriphosphate) (ATP-$\gamma$S) is caused to react with the substrate to introduce mono-thiophosphoric acid group to serine or threonine residue of the substrate protein, substrate protein is labeled by causing a labeling fluorescent material or labeling enzyme to bind to sulfur atom of the mono-thiophosphoric acid group being introduced, amount of labeling of the labeled thiophosphoric acid substrate (amount of fluorescence when labeling fluorescent material is used) is measured, and quantification is made based on the calibration curve prepared by the standard preparation.

The sample to be used for activity measurement is prepared by specifically collecting a target CDK from lysate of a tissue containing malignant tumor that is used as a measurement object. The target CDK can be collected using, for example, anti-CDK antibody.

Expression level of CDK is the amount of the target CDK (unit corresponding to number of molecules) contained in the lysate of the tissue containing malignant tumors which are judgment object, and can be measured by a known method for measuring the amount of target CDK from above-mentioned lysate. For example, the amount of CDK may be measured using ELISA, western blot method, or the like or can be measured by the method disclosed in US2004-0214180. The target CDK is identified and/or isolated using a specific antigen (anti-CDK antibody) and the CDK thus identified and/or isolated is simply quantified.

(Reduction of Tumor)

The major focus of the assays according to the invention is to determine the risk score of a patient's tumor in vitro with the specific assay in order to determine for a patient that can potentially be treated with neoadjuvant therapy whether one can predict before treatment, the potential responsiveness. If this can be achieved, then a patient who is non-responsive in the assay to a single or multiple drug combination, should not be given neoadjuvant therapy. On the other hand, if the patient is significantly responsive to some degree, then the patient should be given the drug or drug combination. The invention would save unnecessary treatment of cancer patients with specific drugs and at the same time define drugs that are likely to work in vivo on the patients' tumor. This is highly applicable to cancer patients that are treated with neoadjuvant therapy such as breast cancer, melanoma, colon, gastric, lung, prostate cancer. Breast cancer being one of the cancers most often treated with neoadjuvant therapy is the primary target but the invention is not limited thereto. Currently, neoadjuvant therapy of breast cancer involves giving treatment of a single or multiple drugs and waiting and seeing if tumor reduction occurs.

(Cell cycle profile score)

It is considered that, even if CDK activated in a certain cell cycle are less, cells are able to proliferate while other specific CDKs act in compensation fashion, and hence, even if specific CDKs are activated abnormally, it may be considered that cell kinetic is already in abnormal state.

According to the present invention, CDK1 specific activity (CDK1 S/A) is considered to be a first risk factor, CDK specific activity ratio (CDK2 specific activity/CDK1 specific activity) to be a second risk factor, and the possible relationship between the first and the second risk factors, and cancer recurrence risk is assessed, respectively. Here, it is supposed that the probability of cancer recurrence has a numerical assessment scale of risk score RS1 based on CDK1 specific activity (first risk factor). Further, it is supposed that the probability of cancer recurrence has a numerical assessment scale of risk score RS2 based on CDK specific activity ratio (second risk factor). If the probability of cancer recurrence by the first and the second risks is represented by cell cycle profile score C2PS, this C2PS is given by a value proportional to a product of the risk score RS1 and risk score RS2.

Cell cycle profile score C2PS, risk score RS1 and risk score RS2 are obtained as follows:

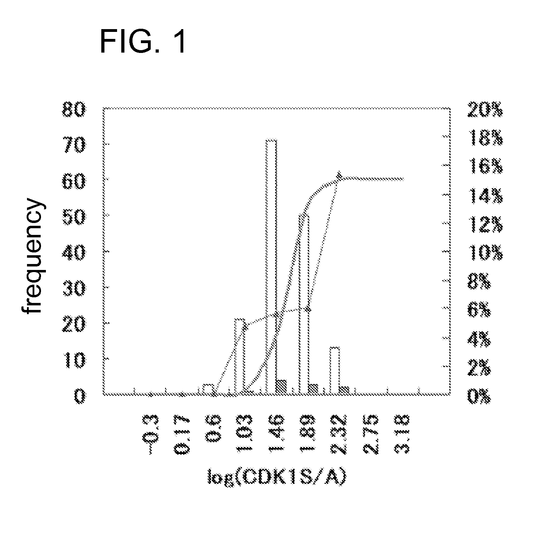

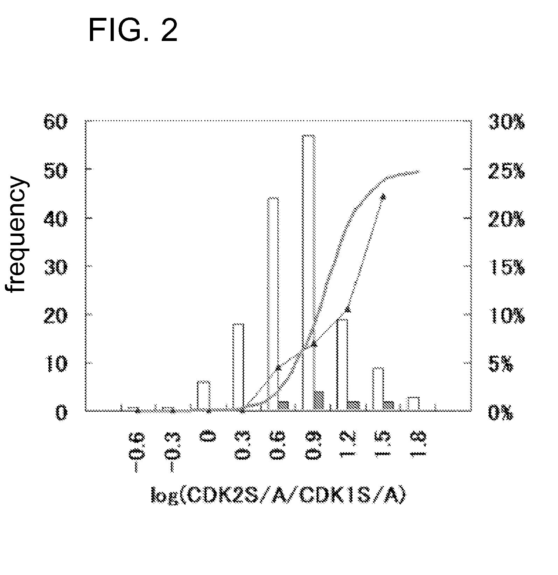

FIG. 1 is a histogram showing the relationship between specific activity of CDK1 (CDK1S/A) obtained from a plurality of breast cancer patients and recurrence rate of the breast cancer. FIG. 2 is a histogram showing relationship between the specific activity ratio of CDK1 and CDK2 [(CDK2S/A)/(CDK1S/A)] obtained from a plurality of breast cancer patients and recurrence rate of the breast cancer. In FIG. 1, the plurality of breast cancer patients are classified based on CDK1 specific activity obtained from these patients, and total number of cases contained in each class is shown by white bars and total number of recurrence cases contained in each class is shown by shaded bars. In FIG. 2, the plurality of breast cancer patients are classified based on CDK2 specific activity/CDK1 specific activity obtained in these patients, and total number of cases contained in each class is shown by white bars and total number of recurrence cases contained in each class is shown by shaded bars. Further in FIGS. 1 and 2, recurrence probability of cancers (number of recurrence cases/total of cases) in each class is obtained and shown by polygonal lines.

In FIG. 1, risk score RS1 in which CDK1 specific activity is considered to be the risk factor can be obtained by approximation of recurrence probability of cancers in each class. Further, in FIG. 2, risk score RS2 in which CDK2 specific activity/CDK1 specific activity is considered to be the risk factor can be obtained by approximation of recurrence probability of cancers in each class. In other words, if recurrence probability of cancers in each class is approximated by logistic functions, in which CDK1 specific activity is represented by x, CDK2 specific activity/CDK1 specific activity is represented by y, risk score RS1 and risk score RS2 can be obtained. Therefore, cell cycle profile score C2PS, risk score RS1 and risk score RS2 are expressed by the following expressions (1) through (3), respectively.

$$C2PS = RS1 \times RS2 = F(x) \times G(y) \qquad (1)$$

$$RS1 = F(x) = a/(1 + Exp(-(x-b) \times c)) \qquad (2)$$

$$RS2 = G(y) = d/(1 + Exp(-(y-e) \times f)) \qquad (3)$$

(in the equation, a through f are constants)

Meanwhile, constants a through c are determined by the relationship between CDK1 specific activity and recurrence probability, and constants d through f are determined by the relationship between CDK2 specific activity/CDK1 specific activity and recurrence probability. For above-shown logistic functions in FIG. 1 and FIG. 2, a = 0.15, b = 1.6, c = 7, d = 0.25, e = 1.0, f = 6.

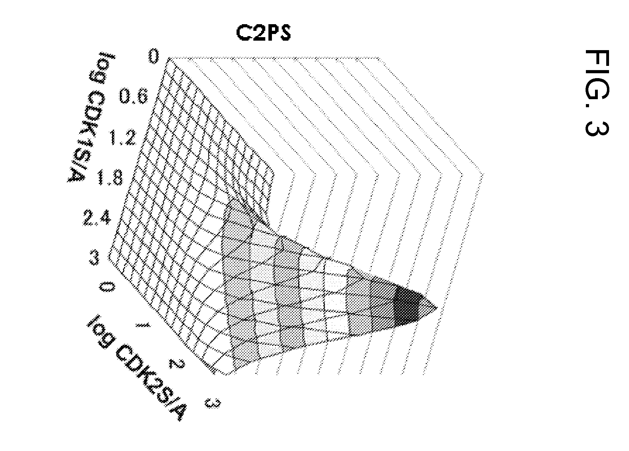

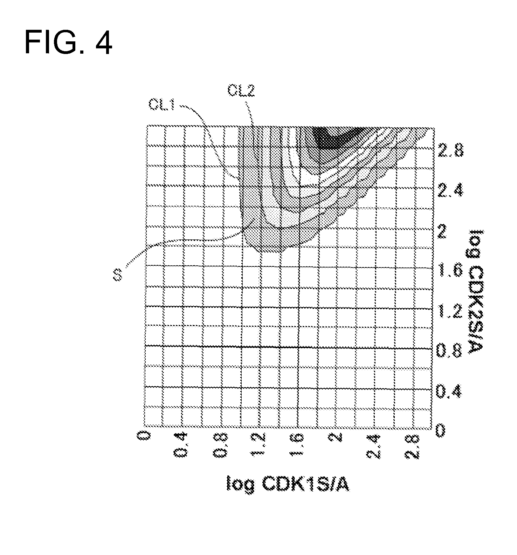

FIG. 3 and FIG. 4 are drawings to schematically explain the cell cycle profile score C2PS. FIG. 3 is a 3-dimensional graph using parameters of logarithm of specific activity of CDK1 (x-axis), logarithm of specific activity of CDK2 (y-axis), and cell cycle profile score C2PS (z-axis), while FIG. 4 is a graph in which cell cycle profile score C2PS (z-axis) shown in FIG. 3 is projected on 2-dimensional surface.

As shown in FIG. 3, if the cell cycle profile score C2PS is projected on 2-dimensional graph, a plurality of curves in contour shape are drawn by combining points where the cell cycle profile scores C2PS are identical.

For example, all points on curve CL1 are points of CDK1 specific activity and CDK2 specific activity where the cell cycle profile scores C2PS are identical, and all points on curve CL2 are points of CDK1 specific activity and CDK2 specific activity where the cell cycle profile scores C2PS are identical. Since an area enclosed by the curves is the area where cell cycle profile score C2PS is always higher than the points on the curve (see FIG. 3), if, for example, the curve CL1 is drawn by combining points of the cell cycle profile score C2PS = M, all areas enclosed by the curve CL1 become such areas where cell cycle profile C2PS >= M. Further, when the curve CL1 is drawn by combining points where the cell cycle profile score C2PS becomes a predetermined value M, and the curve CL2 is drawn by combining points where the cell cycle profile score C2PS becomes a predetermined value N, area S enclosed by the curve CL1 and the curve CL2 is the area where M<= Cell cycle profile score C2PS < N.

As discussed above, a 2-dimensional graph composed of a first CDK specific activity (x-axis) and a second CDK specific activity (y-axis) can be divided into areas each having a different cell cycle profile score C2PS by a curve (cut-off line) combining points where the cell cycle profile scores C2PS are identical.

(Relationship between cell cycle profile score and reduction rate of tumor)

Next, CDK1 expression level, CDK1 active value, CDK2 expression level, CDK2 active value of malignant tumors collected by needle biopsy from breast cancer patients before receiving chemotherapy are measured, and CDK1 specific activity and CDK2 specific activity are obtained. The cell cycle profile score is obtained by above-shown equations and results are shown in Table 1. Reduction rates of tumors of each of the patients after chemotherapy are also shown in Table 1. Meanwhile, the reduction rate of tumor is obtained by measuring the size of the tumor tissue of each patient before and after the chemotherapy.

Table 1

| Patient | CDK1S A | CDK2S A | C2PS | Reduction rate of tumor | Regimen |
|---|---|---|---|---|---|
| 1 | 56.47 | 904.40 | 0.004 | 16 | wT-FEC |
| 2 | 48.38 | 373.42 | 0.007 | 45 | wT-FEC |
| 3 | 39.43 | 298.49 | 0.020 | 58 | CE-Doce |
| 4 | 16.11 | 545.31 | 0.028 | 20 | CE-Doce |
| 5 | 125.88 | 614.91 | 0.038 | 44 | wT-FEC |
| 6 | 90.36 | 3899.25 | 0.209 | 83 | CE |
| 7 | 17.68 | 265.96 | 0.376 | 51 | wT-FEC |
| 8 | 17.96 | 164.54 | 0.396 | 68 | wT-FEC |
| 9 | 41.09 | 190.27 | 0.522 | 100 | wT-FEC |
| 10 | 90.98 | 18.48 | 0.550 | 90 | wT-FEC |
| 11 | 7.29 | 339.58 | 0.599 | 100 | wT-FEC |
| 12 | 76.64 | 313.75 | 0.630 | 100 | CE-Doce |
| 13 | 61.57 | 167.28 | 0.679 | 49 | wT-FEC |
| 14 | 13.28 | 218.80 | 0.933 | 90 | CE-Doce |
| 15 | 2.33 | 177.24 | 1.352 | 75 | wT-FEC |
| 16 | 190.31 | 282.99 | 1.544 | 82 | CE-Doce |
| 17 | 37.55 | 99.07 | 2.883 | 74 | wT-FEC |
| 18 | 80.68 | 497.45 | 2.966 | 87 | wT-FEC |
| 19 | 8.87 | 21.51 | 3.022 | 87 | wT-FEC |

(continued)

| Patient | CDK1S A | CDK2S A | C2PS | Reduction rate of tumor | Regimen |
|---|---|---|---|---|---|
| 20 | 59.39 | 647.84 | 3.032 | 60 | CE+ZL,TM |
| 21 | 11.27 | 95.32 | 4.071 | 85 | wT-FEC |
| 22 | 11.33 | 69.15 | 6.423 | 87 | wT-FEC |
| 23 | 20.77 | 63.60 | 6.486 | 100 | CE-Doce |
| 24 | 23.00 | 61.21 | 6.533 | 79 | CE-Doce |
| 25 | 10.21 | 61.82 | 6.989 | 70 | wT |
| 26 | 17.14 | 89.82 | 8.799 | 100 | wT-FEC |
| 27 | 58.70 | 223.36 | 22.259 | 95 | wT-FEC |
| 28 | 9.38 | 500.49 | 45.372 | 95 | wT-FEC |
| 29 | 6.24 | 349.89 | 48.284 | 88 | CE+UFT |
| 30 | 40.87 | 657.88 | 51.233 | 72 | CE-Doce |
| 31 | 31.85 | 68.54 | 64.661 | 56 | CE-Doce |
| 32 | 12.49 | 354.67 | 101.649 | 92 | CE-Doce |

In Table 1, CDK1SA denotes CDK1 specific activity, CDK2SA denotes CDK2 specific activity, C2PS denotes cell cycle profile score, and reduction rate of tumor denotes reduction rate of tumors after receiving the chemotherapy prescribed in the regimen. In the column of the regimen, "wT" means paclitaxel, "FEC" means fluorouracil, cyclophosphamide, epirubicin, "CE" means cyclophosphamide, epirubicin, "Doce" means docetaxel, "ZL" means zoladex, "TM" means tamoxifen, and "UFT" means uracil, tegafur.

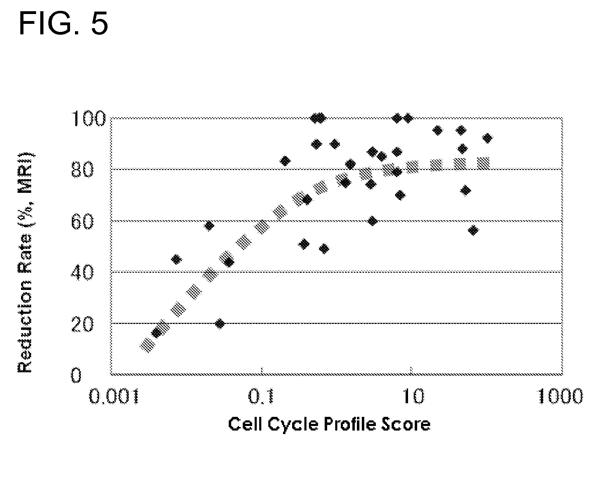

Results of investigation of a relationship between the cell cycle profile score C2PS and reduction rate of tumors after receiving the chemotherapy revealed that there is a correlation between the two as shown in FIG. 5.

(Relationship between cell cycle profile score and response rate for reduction of tumor)

In Table 1, patients are listed in the order of increasing cell cycle profile scores. They are grouped so that 4 to 6 patients with similar (or smaller) cell cycle profile scores may be in the same group. Specifically, patients 1 to 4 are assigned to a first group, patients 1 to 5 are assigned to a second group, patients 1 to 6 are assigned to a third group, patients 2 to 7 are assigned to a fourth group, and after that, groups consisting of 6 patients are formed by shifting one patient. Next, mean value of cell cycle profile scores, frequency of appearance of patients with reduction rate of tumor greater than 50%, and frequency of appearance of patients with reduction rate of tumor greater than 80% are calculated for each of the groups. Results are shown in Table 2.

Table 2

| Group | Patients | Mean value of C2PS | Frequency of reduction rate greater than 50% | Frequency of reduction rate greater than 80% |
|---|---|---|---|---|
| 1 | 1-4 | 0.015 | 25% | 0 |
| 2 | 1-5 | 0.019 | 20% | 0 |
| 3 | 1-6 | 0.051 | 33.3% | 16.7% |
| 4 | 2-7 | 0.113 | 50% | 16.7% |
| 5 | 3-8 | 0.178 | 66.7% | 16.7% |
| 6 | 4-9 | 0.262 | 66.7% | 33.3% |
| 7 | 5-10 | 0.349 | 83.3% | 50% |
| 8 | 6-11 | 0.442 | 100% | 66.7% |
| 9 | 7-12 | 0.512 | 100% | 66.7% |

(continued)

| Group | Patients | Mean value of C2PS | Frequency of reduction rate greater than 50% | Frequency of reduction rate greater than 80% |
|---|---|---|---|---|
| 10 | 8-13 | 0.563 | 83.3% | 66.7% |
| 11 | 9-14 | 0.652 | 83.3% | 83.3% |
| 12 | 10-15 | 0.791 | 83.3% | 66.7% |
| 13 | 11-16 | 0.956 | 83.3% | 66.7% |
| 14 | 12-17 | 1.337 | 83.3% | 50% |
| 15 | 13-18 | 1.726 | 83.3% | 50% |
| 16 | 14-19 | 2.117 | 100% | 66.7% |
| 17 | 15-20 | 2.467 | 100% | 50% |
| 18 | 16-21 | 2.920 | 100% | 66.7% |
| 19 | 17-22 | 3.733 | 100% | 66.7% |
| 20 | 18-23 | 4.333 | 100% | 83.3% |
| 21 | 19-24 | 4.928 | 100% | 66.7% |
| 22 | 20-25 | 5.589 | 100% | 50% |
| 23 | 21-26 | 6.550 | 100% | 66.7% |
| 24 | 22-27 | 9.582 | 100% | 66.7% |
| 25 | 23-28 | 16.073 | 100% | 66.7% |
| 26 | 24-29 | 23.039 | 100% | 66.7% |
| 27 | 25-30 | 30.489 | 100% | 66.7% |
| 28 | 26-31 | 40.101 | 100% | 66.7% |
| 29 | 27-32 | 55.576 | 100% | 66.7% |

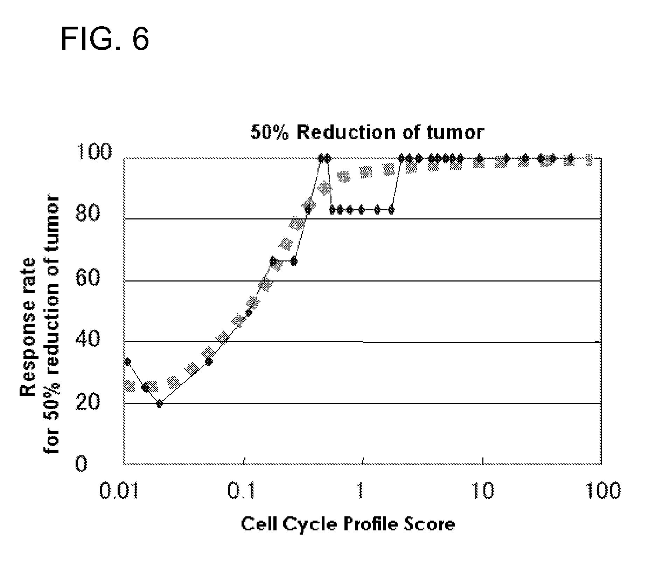

A graph is drawn based on results shown in Table 2, in which graph horizontal axis represents mean value of cell cycle profile scores and vertical axis represents frequency of appearance (reduction rate > 50%). As shown in FIG. 6, there is a predetermined correlation between the mean value of cell cycle profile scores and the frequency of appearance (reduction rate > 50%), and a logistic curve can then be drawn. Using the graph thus obtained, it is now possible to predict, for example, "How much possibility can a patient with cell cycle profile score X expect a reduction rate of tumor more than 50% by the chemotherapy?" That is, it is possible to predict a degree of response of the chemotherapy based on the cell cycle profile score. Thus, the degree of response of the chemotherapy obtained from the cell cycle profile score presents physicians a useful index for judgment whether the chemotherapy should be given to patients. Further, for breast cancer case, reduction rate of tumor more than 50% is used as an index whether or not preoperative chemotherapy for the sake of breast conservation should be provided. Thus, the cell cycle profile score is a useful index to make a judgment whether or not the preoperative chemotherapy should be given.

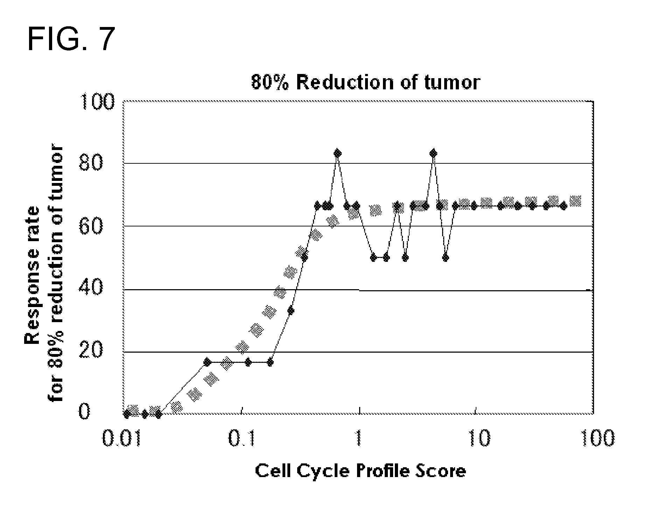

Subsequently, as shown in FIG. 7, a graph is drawn based on the results shown in Table 2 in which the graph horizontal axis represents the mean value of cell cycle profile scores and the vertical axis represents frequency of appearance (reduction rate > 80%). As shown in FIG. 7, there is a predetermined correlation between the mean value of cell cycle profile scores and frequency of appearance (reduction rate > 80%), and a logistic curve can then be drawn. With the use of the graph thus obtained, it is now possible to predict, for example, "What is the probability that a patient with cell cycle profile score X can expect a reduction rate of tumor by more than 80% by chemotherapy?". That is, it is possible to predict a degree of response of the chemotherapy based on the cell cycle profile score. Thus, the degree of response of the chemotherapy obtained from the cell cycle profile score presents physicians a useful index for judgment whether or not the chemotherapy should be given to patients. Further, reduction rate of tumor more than 80% is used as an index for judgment whether or not the tumor can be completely cured by administrating the chemotherapy.

(Relationship between cell cycle profile score and pathological complete response)

For breast cancer patients listed in Table 3 shown below, CDK1 expression level, CDK1 active value, CDK2 expression level, CDK2 active value of malignant tumors collected by needle biopsy before receiving chemotherapy are measured, and CDK1 specific activity and CDK2 specific activity are obtained. Cell cycle profile scores are obtained based on above-shown equations and results are shown in Table 3. Further, for each of patients shown in Table 3, whether or not pathological complete response (pCR) has been attained by the chemotherapy is shown in Table 3. In the column "pCR" in Table 3, "1" denotes pathological complete response.

Table 3

| Patients | CDK1SA | CDK2SA | C2PS | pCR | Regimen |
|---|---|---|---|---|---|
| 1 | 56.47 | 904.40 | 0.004 | 0 | wT-FEC |
| 2 | 48.38 | 373.42 | 0.007 | 0 | wT-FEC |
| 5 | 125.88 | 614.91 | 0.038 | 0 | wT-FEC |
| 7 | 17.68 | 265.96 | 0.376 | 0 | wT-FEC |
| 8 | 17.96 | 164.54 | 0.396 | 0 | wT-FEC |
| 9 | 41.09 | 190.27 | 0.522 | 1 | wT-FEC |
| 10 | 90.98 | 18.48 | 0.550 | 0 | wT-FEC |
| 11 | 7.29 | 339.58 | 0.599 | 1 | wT-FEC |
| 13 | 61.57 | 167.28 | 0.679 | 0 | wT-FEC |
| 15 | 2.33 | 177.24 | 1.352 | 0 | wT-FEC |
| 33 | 22.25 | 101.43 | 1.875 | 0 | wT-FEC |
| 17 | 37.55 | 99.07 | 2.883 | 0 | wT-FEC |
| 18 | 80.68 | 497.45 | 2.966 | 1 | wT-FEC |
| 19 | 8.87 | 21.51 | 3.022 | 0 | wT-FEC |
| 34 | 213.15 | 580.1 | 3.643 | 1 | wT-FEC |
| 21 | 11.27 | 95.32 | 4.071 | 0 | wT-FEC |
| 35 | 22.89 | 162.64 | 5.132 | 1 | wT-FEC |
| 36 | 15.26 | 595.97 | 5.624 | 0 | wT |
| 22 | 11.33 | 69.15 | 6.423 | 0 | wT-FEC |
| 25 | 10.21 | 61.82 | 6.989 | 0 | wT |
| 26 | 17.14 | 89.82 | 8.799 | 1 | wT-FEC |
| 27 | 58.70 | 223.36 | 22.259 | 0 | wT-FEC |
| 28 | 9.38 | 500.49 | 45.372 | 1 | wT-FEC |

Patients listed in Table 3 are grouped in the order of smaller cell cycle profile scores and shown in Table 4. Next, the mean value of cell cycle profile scores and pCR rate are calculated for every group. Results are shown in Table 4.

Table 4

| Group | Patients | Mean value of C2PS | pCR rate |
|---|---|---|---|
| 1 | 1,2,5,7 | 0.106 | 0 |
| 2 | 1,2,5,7,8 | 0.164 | 0 |
| 3 | 1,2,5,7-9 | 0.224 | 16.7 |
| 4 | 2,5,7-10 | 0.315 | 16.7 |
| 5 | 5,7-11 | 0.413 | 33.3 |
| 6 | 7-11,13 | 0.520 | 33.3 |
| 7 | 8-11,13,15 | 0.683 | 33.3 |

(continued)

| Group | Patients | Mean value of C2PS | pCR rate |
|-------|----------|-------------------|----------|
| 8 | 9-11,13,15,33 | 0.930 | 33.3 |
| 9 | 10,11,13,15,33,17 | 1.323 | 16.7 |
| 10 | 11,13,15,33,17,18 | 1.726 | 33.3 |
| 11 | 13,15,33,17-19 | 2.130 | 16.7 |
| 12 | 15,33,17-19,34 | 2.624 | 33.3 |
| 13 | 33,17-19,34,21 | 3.077 | 33.3 |
| 14 | 17-19,34,21,35 | 3.619 | 50 |
| 15 | 18,19,34,21,35,36 | 4.076 | 50 |
| 16 | 19,34,21,35,36,22 | 4.652 | 33.3 |
| 17 | 34,21,35,36,22,25 | 5.314 | 33.3 |
| 18 | 21,35,36,22,25,26 | 6.173 | 33.3 |
| 19 | 35,36,22,25-27 | 9.204 | 33.3 |
| 20 | 36,22,25-28 | 15.911 | 33.3 |

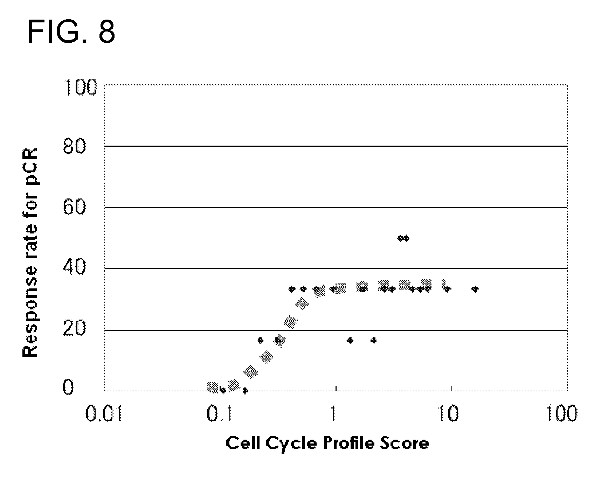

A graph is drawn based on results shown in Table 4, in which the graph horizontal axis represents the mean value of cell cycle profile scores and the vertical axis represents the pCR rate, and is shown in FIG. 8. As shown in FIG. 8, there is a predetermined correlation between mean value of cell cycle profile scores and pCR rate, and a logistic curve can then be drawn. It has been revealed that the pCR rate can be predicted from cell cycle profile score based on such correlative relationship.

The method according to the embodiments of the invention is preferably executed by a computer system. The computer system for executing the method is described below.

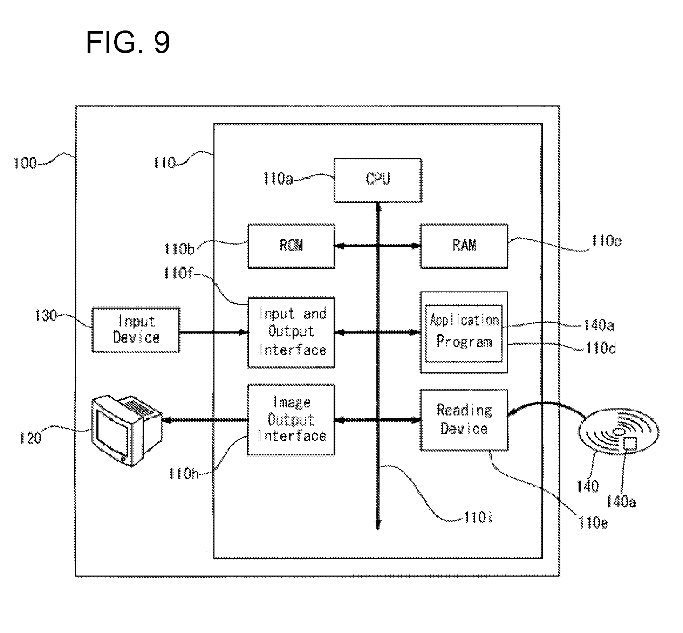

FIG. 9 is a block diagram showing a computer system (also referred to as "system" hereinafter), in accordance with an embodiment of the invention. A system 100 related to the present embodiment is composed of a main body 110, a display 120, and an input device 130. The main body 110 comprises a CPU 110a, a ROM 110b, a RAM 110c, a hard disk 110d, a readout device 110e, an input/output interface 110f, and an image output interface 110h. The CPU 110a, the ROM 110b, the RAM 110c, the hard disk 110d, the readout device 110e, the input/output interface 110f, and the image output interface 110h are data-communicably connected by a bus 110i.

The CPU 110a is capable of executing a computer program recorded in the ROM 110b and a computer program loaded in the RAM 110c. The ROM 110b comprises mask ROM, PROM, EPROM, EEPROM, etc. and is recoded with computer programs executed by the CPU 110a and data used for the programs.

The RAM 110c comprises SRAM, DRAM, etc. The RAM 110c is used to read out computer programs recorded in the ROM 110b and the hard disk 110d. And the RAM 110c is used as a work area of the CPU 110a when these computer programs are executed.

The hard disk 110d is installed with an operating system, an application program, etc., various computer programs to be executed by the CPU 110a, and data used for executing the computer programs. An application program 140a described later is also installed in this hard disk 110d.

The readout device 110e which comprises a flexible disk drive, a CD-ROM drive or DVD-ROM drive is capable of reading out a computer program or data recorded in a portable recording media 140. And the portable recording media 140 stores the application program 140a to function as a system of the present invention. The computer 100a reads out the application program 140a related to the present invention from the portable recording media 140 and is capable of installing the application program 140a in the hard disk 110d.

In addition to that said application program 140a is provided by the portable recording media 140, said application program 140a may be provided through an electric communication line (wired or wireless) from outside devices which are communicably connected to the computer 100a via said electric communication line. For example, said application program 140a is stored in a hard disk in an internet server computer to which the computer 100a accesses and said application program 140a may be downloaded and installed in the hard disk 110d.

The hard disk 110d is installed with an operating system which provides a graphical user interface environment, such as that sold under the trademark WINDOWS by Microsoft Corporation. In the explanation hereinafter, the application program 140a related to this embodiment shall operate on said operating system.

The input/output interface 110f comprises a serial interface, e.g. USB, IEEE1394, RS-232C, etc.; a parallel interface, e.g. SCSI, IDE, IEEE1284, etc.; and an analog interface e.g. D/A converter, A/D converter, etc. The input/output interface 110f is connected to the input device 130 comprising a keyboard and a mouse and users can input data into the computer 100a using the input data device 130.

The image output interface 110h is connected to the display 120 comprising LCD, CRT or the like so that picture signals corresponding to image data provided from the CPU 110a are output to the display 120. The display 120 displays a picture (screen) based on input picture signals.

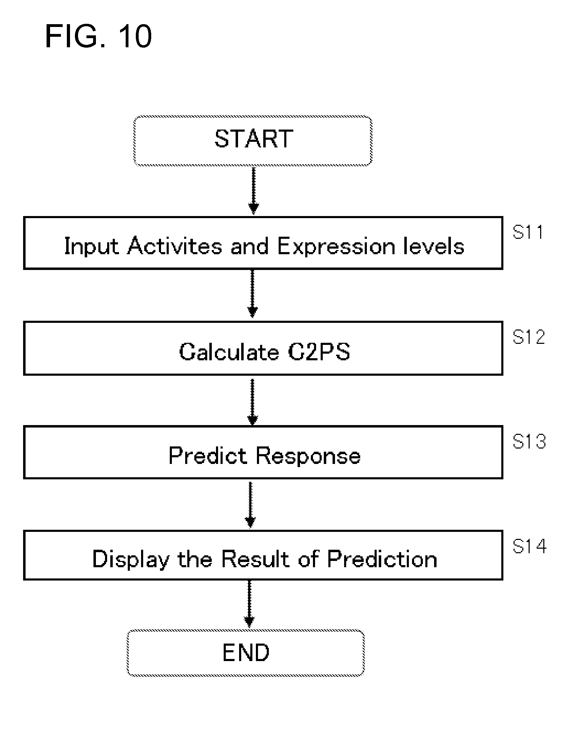

FIG. 10 is a flow chart for performing the method executed by the CPU 110a. When the activities of CDK1 and CDK2 and the expression levels of CDK1 and CDK2of a specimen are inputted from the input unit 130, the CPU 110a obtains these parameter data via the input/output interface 110f and stores the obtained data in a RAM 110c (step S11).

The CPU 110a previously reads the expressions (1) through (3) stored in the hard disk 110d, and calculates a cell cycle profile score C2PS form the input parameter data (Step S12).

Next, the CPU 110a predicts and judges the response of the cancer patient to anticancer drug on the basis of the calculated cell cycle profile score C2PS (Step S13).

A first through fourth correlation equation are stored on the hard disk 110d beforehand. The first correlation equation relates to the correlation between cell cycle profile scores and reduction rates showed by Fig. 5. The second correlation equation relates to the correlation between cell cycle profile scores and response rates for 50% reduction of tumor showed by Fig. 6. The third correlation equation relates to a correlation between cell cycle profile scores and response rates for 80% reduction of tumor showed by Fig. 7. The forth correlation equation relates to a correlation between cell cycle profile scores and response rates for pCR showed by Fig. 8.

The CPU 110a obtains a reduction rate as the response of the cancer patient to anticancer drug on the basis of the calculated cell cycle profile score C2PS and the read out first correlation equation from the hard disk 110d. The CPU 110a obtains a response rate for 50% reduction of tumor as the response of the cancer patient to anticancer drug on the basis of the calculated cell cycle profile score C2PS and the read out second correlation equation from the hard disk 110d. The CPU 110a obtains a response rate for 80% reduction of tumor as the response of the cancer patient to anticancer drug on the basis of the calculated cell cycle profile score C2PS and the read out third correlation equation from the hard disk 110d. The CPU 110a obtains a response rate for pCR as the response of the cancer patient to anticancer drug on the basis of the calculated cell cycle profile score C2PS and the read out fourth correlation equation from the hard disk 110d.

The CPU 110a stores the obtained reduction rate, response rate for 50% reduction of tumor, response rate for 80% reduction of tumor and response rate for pCR in the hard disk 110d and display the reduction rate, response rate for 50% reduction of tumor, response rate for 80% reduction of tumor and/or response rate for pCR on the display 120 through the input/output interface 110f (Step 14).

In particular embodiments of the invention, the activities of CDK1 and CDK2 and the expression levels of CDK1 and CDK2 are inputted via the input device 130; however, the input method is not limited thereto. For example, the data is not necessarily inputted by an operator, and the activities of CDK1 and CDK2 and the expression levels of CDK1 and CDK2 may be automatically obtained from a measuring device via the input/output interface 110f.

**Claims**

1. A method for predicting a response of a cancer patient to anticancer drug, comprising steps of:

   acquiring specific activities of cyclin-dependant kinase 1 (CDK1) and CDK2 contained in a malignant tumor obtained from a cancer patient;
   obtaining a cell cycle profile score based on the specific activity of CDK1 (CDK1 specific activity) and the specific activity of CDK2 (CDK2 specific activity); and
   predicting a degree of response to the anticancer drug based on the cell cycle profile score prior to treatment,

   wherein the CDK1 specific activity is represented by a ratio of an activity value of CDK1 to an expression level of CDK1 and the CDK2 specific activity is represented by a ratio of an activity value of CDK2 to an expression level of CDK2.

2. A method for predicting response of cancer patient to anticancer drug, comprising steps of:

   acquiring specific activities of cyclin-dependant kinase 1 (CDK1) and CDK2 contained in a malignant tumor obtained from a cancer patient;
   obtaining a cell cycle profile score based on the specific activity of CDK1 (CDK1 specific activity) and the specific activity of CDK2 (CDK2 specific activity); and
predicting a degree of tumor reduction based on the cell cycle profile score prior to treatment,

wherein the CDK1 specific activity is represented by a ratio of an activity value of CDK1 to an expression level of CDK1 and the CDK2 specific activity is represented by a ratio of an activity value of CDK2 to an expression level of CDK2.

3. A method for predicting a response of a cancer patient to anticancer drug, comprising steps of:

acquiring specific activities of cyclin-dependant kinase 1 (CDK1) and CDK2 contained in a malignant tumor obtained from a cancer patient;
obtaining a cell cycle profile score based on the specific activity of CDK1 (CDK1 specific activity) and the specific activity of CDK2 (CDK2 specific activity); and
predicting a pathological complete response rate based on the cell cycle profile score prior to treatment,

wherein the CDK1 specific activity is represented by a ratio of an activity value of CDK1 to an expression level of CDK1 and the CDK2 specific activity is represented by a ratio of an activity value of CDK2 to an expression level of CDK2.

4. The method according to any one of claims 1 to 3, wherein the treatment is an anticancer drug treatment for neoadjuvant therapy.

5. The method according to any one of claims 1 to 3, wherein the predicting step is performed by predicting the degree of response to a combination of anticancer drugs.

6. The method according to claim 4, wherein the neoadjuvant therapy is applied to sarcoma, melanoma, and breast, colorectal, lung, gastric, esophageal, pancreas, and/or other carcinomas.

7. The method according to any one of claims 1 to 6, further comprising the steps of performing a second acquiring step, a second obtaining step and a second predicting step post to the treatment.

8. The method according to any one of claims 1 to 3, wherein the treatment is an anticancer drug treatment for adjuvant therapy.

9. The method according to any one of claims 1 to 8, wherein the cell cycle profile score is obtained based on the CDK1 specific activity and a ratio of the CDK2 specific activity to the CDK1 specific activity.

10. The method according to any one of claims 1 to 9, wherein the cell cycle profile score is obtained based on the following expression:

$$\text{Cell cycle profile score} = F(x) \times G(y) \qquad (1)$$

wherein x represents the CDK1 specific activity, y represents the ratio of the CDK2 specific activity to the CDK1 specific activity.

11. The method according to claim 10, wherein the F(x) and G(y) are represented by the following expressions:

$$F(x)=a/(1+Exp(-(x-b)\times c))$$

$$G(y)=d/(1+Exp(-(y-e)\times f))$$

wherein a through f are constants.

# FIG. 1

# FIG. 2

FIG. 3

FIG. 4

FIG. 5

FIG. 6

**50% Reduction of tumor**

# FIG. 7

**80% Reduction of tumor**

FIG. 8

FIG. 9

# FIG. 10

```
┌─────────────────────────────┐
│            START            │
└─────────────────────────────┘
                │
                ▼
┌─────────────────────────────────────┐
│ Input Activites and Expression levels│  S11
└─────────────────────────────────────┘
                │
                ▼
┌─────────────────────────────────────┐
│           Calculate C2PS             │  S12
└─────────────────────────────────────┘
                │
                ▼
┌─────────────────────────────────────┐
│          Predict Response            │  S13
└─────────────────────────────────────┘
                │
                ▼
┌─────────────────────────────────────┐
│    Display the Result of Prediction  │  S14
└─────────────────────────────────────┘
                │
                ▼
┌─────────────────────────────┐
│             END             │
└─────────────────────────────┘
```

Europäisches Patentamt
European Patent Office
Office européen des brevets

# EUROPEAN SEARCH REPORT

**Application Number**

EP 09 16 8995

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 767 647 A (SYSMEX CORP [JP]) 28 March 2007 (2007-03-28) * page 3, paragraphs 14, 18, 19 * * page 9, paragraphs 62,63 * * page 18, paragraph 118; claims 1-17 * | 1-11 | INV. G01N33/50 G01N33/574 |
| X | KIM S J ET AL: "Determination of the specific activity of CDK1 and CDK2 as a novel prognostic indicator for early breast cancer." ANNALS OF ONCOLOGY : OFFICIAL JOURNAL OF THE EUROPEAN SOCIETY FOR MEDICAL ONCOLOGY / ESMO JAN 2008, vol. 19, no. 1, January 2008 (2008-01), pages 68-72, XP002552410 ISSN: 1569-8041 * abstract * * page 69, left-hand column, paragraphs 2,3 * * page 70, left-hand column, paragraph 1 - right-hand column, paragraph 1 * * table 1 * * page 71, left-hand column, paragraph 2 * * page 72, left-hand column, paragraph 1-3 * | 1-11 | |
| X | EP 1 770 170 A (SYSMEX CORP [JP]) 4 April 2007 (2007-04-04) * pages 24-25, paragraph 145 * * table 9 * | 1-11 | |
| X | EP 1 739 429 A (SYSMEX CORP [JP]) 3 January 2007 (2007-01-03) * claims 1-14 * | 1-11 | |

TECHNICAL FIELDS SEARCHED (IPC)

G01N

-/--

The present search report has been drawn up for all claims

| Place of search | Date of completion of the search | Examiner |
|---|---|---|
| Munich | 27 October 2009 | Pilch, Bartosz |

EP 2 163 894 A1

Europäisches
Patentamt

European
Patent Office

Office européen
des brevets

**EUROPEAN SEARCH REPORT**

Application Number

EP 09 16 8995

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | UENO NAOTO T ET AL: "CYCLIN-DEPENDENT KINASE (CDK) PROFILING TO PREDICT PACLITAXEL CHEMOSENSITIVITY" PROCEEDINGS OF THE ANNUAL MEETING OF THE AMERICAN ASSOCIATION FOR CANCER RESEARCH, NEW YORK, NY, US, vol. 46, 1 April 2005 (2005-04-01), page 107, XP001536581 ISSN: 0197-016X * abstract * | 1-11 | |
| A | PAYTON MARC ET AL: "Discovery and evaluation of dual CDK1 and CDK2 inhibitors." CANCER RESEARCH 15 APR 2006, vol. 66, no. 8, 15 April 2006 (2006-04-15), pages 4299-4308, XP002552411 ISSN: 0008-5472 * the whole document * | 1-11 | |
| A | CAI DONGPO ET AL: "Combined depletion of cell cycle and transcriptional cyclin-dependent kinase activities induces apoptosis in cancer cells." CANCER RESEARCH 15 SEP 2006, vol. 66, no. 18, 15 September 2006 (2006-09-15), pages 9270-9280, XP002552412 ISSN: 0008-5472 * the whole document * | 1-11 | TECHNICAL FIELDS SEARCHED (IPC) |

The present search report has been drawn up for all claims

| Place of search | Date of completion of the search | Examiner |
|---|---|---|
| Munich | 27 October 2009 | Pilch, Bartosz |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 09 16 8995

This annex lists the patent family members relating to the patent documents cited in the above-mentioned European search report.
The members are as contained in the European Patent Office EDP file on
The European Patent Office is in no way liable for these particulars which are merely given for the purpose of information.

27-10-2009

| Patent document cited in search report | | Publication date | Patent family member(s) | | Publication date |
|---|---|---|---|---|---|
| EP 1767647 | A | 28-03-2007 | WO | 2005116241 A1 | 08-12-2005 |
| | | | US | 2007231837 A1 | 04-10-2007 |
| EP 1770170 | A | 04-04-2007 | CN | 1971276 A | 30-05-2007 |
| | | | JP | 2007097448 A | 19-04-2007 |
| | | | US | 2007077601 A1 | 05-04-2007 |
| EP 1739429 | A | 03-01-2007 | US | 2007003964 A1 | 04-01-2007 |

For more details about this annex : see Official Journal of the European Patent Office, No. 12/82

**REFERENCES CITED IN THE DESCRIPTION**

**Patent documents cited in the description**

- US 20020164673 A
- US 20040214180 A